# EUROPEAN PATENT SPECIFICATION

(11) **EP 2 582 822 B1**
(45) Date of publication and mention of the grant of the patent: **06.05.2015**
(21) Application number: 11728477.8
(22) Date of filing: 16.06.2011
(51) Int. Cl.: C12P 7/10

(54) **Method for producing ethanol from biomass**
Verfahren zur Herstellung von Ethanol aus Biomasse
Procédé de production d'éthanol à partir de biomasse

(30) Priority: 17.06.2010 US 355922 P
(43) Date of publication of application: 24.04.2013
(73) Proprietor: POET Research, Inc., Sioux Falls, South Dakota 57104 (US)
(72) Inventor: NARENDRANATH, Neelakantam, V., Sioux Falls North Dakota 57108 (US)
(74) Representative: Arth, Hans-Lothar
(86) International application number: PCT/US2011/040726
(87) International publication number: WO 2011/159915

(56) References cited:
- WO-A-2010/102063
- US-A- 4 368 268
- SONG ET AL: "Issues with increasing bioethanol productivity: A model directed study", KOREAN JOURNAL OF CHEMICAL ENGINEERING, vol. 27, 2010, pages 576-586, XP000002657834,
- KIM ET AL: "Effect of compositional variability of distillers' grains on cellulosic ethanol production", BIORESOURCE TECHNOLOGY, vol. 101, 12 March 2010 (2010-03-12), pages 5385-5393, XP000002657835,
- KUYPER ET AL: "Evolutionary engineering of mixed-sugar utilization by a xylose-fermenting Saccharomyces cerevisiae strain", FEMS YEAST RESEARCH, vol. 5, 2005, pages 925-934, XP004967640,
- VAN MARIS ET AL: "Alcoholic fermentation of carbon sources in biomass hydrolysates by Saccharomyces cerevisiae: current status", ANTONIE VAN LEEUWENHOEK, vol. 90, 2006, pages 391-418, XP019446684,
- NAKATA ET AL: "Ethanol production with beta-xylosidase, xylose isomerase, and Saccharomyces cerevisiae from the hydrolysate of Japanese beech after hot-compressed water treatment", JOURNAL OF WOOD SCIENCE, vol. 55, 2009, pages 289-294, XP019724960,
- TOMÁS-PEJÓ ET AL: "Adaptation of the xylose fermenting yeast Saccharomyces cerevisiae F12 for improving ethanol production in different fed-batch SSF processes", JOURNAL OF INDUSTRIAL MICROBIOLOGY AND BIOTECHNOLOGY, vol. 37, 29 June 2010 (2010-06-29), pages 1211-1220, XP019809596,
- LIU ET AL: "gTME for improved adaptation of Saccharomyces cerevisiae to corn cob acid hydrolysate", APPLIED BIOCHEMISTRY AND BIOTECHNOLOGY, vol. 164, 2 March 2011 (2011-03-02), pages 1150-1159, XP019918043,

## Description

### FIELD

The subject disclosure relates to a system for fermentation of biomass to be used in the production of ethanol.

### BACKGROUND

Ethanol can be produced from grain-based feedstocks (e.g. corn, sorghum/milo, barley, wheat, soybeans, etc.), from sugar (e.g. from sugar cane, sugar beets, etc.), and from biomass (e.g. from cellulosic feedstocks such as switchgrass, corn cobs and stover, wood or other plant material).

Biomass comprises plant matter that can be suitable for direct use as a fuel/energy source or as a feedstock for processing into another bioproduct (e.g., a biofuel such as cellulosic ethanol) produced at a biorefinery (such as an ethanol plant). Biomass may comprise, for example, corn cobs and stover (e.g., stalks and leaves) made available during and/or after harvesting of the corn kernels, fiber from the corn kernel, switchgrass, farm or agricultural residue, wood chips or other wood waste, and other plant matter. In order to be used or processed, biomass will be harvested and collected from the field and transported to the location where it is to be used or processed.

In a biorefinery configured to produce ethanol from biomass such as cellulosic feedstocks, ethanol is produced from lignocellulosic material (e.g. cellulose and/or hemi-cellulose). The biomass is prepared so that sugars in the cellulosic material (such as glucose from the cellulose and xylose from the hemi-cellulose) can be accessed and fermented into a fermentation product that comprises ethanol (among other things). The fermentation product is then sent to the distillation system, where the ethanol is recovered by distillation and dehydration. Other bioproducts, such as lignin and organic acids, may also be recovered as co-products. Determination of how to more efficiently prepare and treat the biomass for production into ethanol will depend upon (among other things) the form and type or composition of the biomass.

It would be advantageous to provide for a system and method for fermenting a liquid component of pre-treated biomass. It would also be advantageous to provide for a system and method for fermenting a liquid component of pre-treated biomass in a fed batch fermentation that utilizes a low yeast dose. It would further be advantageous to provide for a system that provides one or more features to facilitate improvement in the efficiency and yield of cellulosic ethanol from biomass.

### SUMMARY

The subject disclosure relates to a method for producing a fermentation product in a fermentation system from biomass that has been pre-treated and separated into a first component and a second component. The method comprises preparing a slurry comprising: supplying the first component to the fermentation system and providing an ethanologen to the fermentation system. The method also comprises adjusting the pH of the slurry to a range of about 4.5 to about 6.5, maintaining the first component and the ethanologen in the fermentation system at a temperature of between about 25 and about 37 degrees Celsius, and recovering the fermentation product from the fermentation system. The ethanologen is supplied to the fermentation system in a concentration of less than about 2 grams of ethanologen on a dry basis per liter of slurry. The biomass comprises lignocellulosic material. The first component comprises pentose. The pentose comprises xylose. The ethanologen is capable of fermenting xylose into ethanol.

The subject disclosure also relates to a method for producing a fermentation product in a fermentation system from lignocellulosic biomass that has been pre-treated and separated into a first component and a second component. The method comprises: preparing a slurry comprising: supplying the first component to the fermentation system and providing an ethanologen to the fermentation system in a concentration of less than about 2 grams of ethanologen on a dry basis per liter of slurry. The ethanologen is capable of fermenting xylose into ethanol. The method also comprises adjusting the pH of the slurry to a range of about 4.5 to about 6.5, maintaining the first component and the ethanologen in the fermentation system at a temperature of between about 25 and about 37 degrees Celsius for a duration of at least about 48 hours, and supplying an additional amount of the first component to the slurry. The first component comprises xylose and the supply of the additional amount of the first component begins at least about 5 hours but no more than about 40 hours after providing the ethanologen to the slurry. The additional amount of the first component is supplied to the slurry at a rate of about 2 to about 5 grams of xylose added per liter of slurry per hour. The total amount of xylose added to the slurry is between about 110 to about 140 grams per liter of slurry. The method also includes recovering the fermentation product from the fermentation system. The fermentation product comprises ethanol, and wherein at least about 75 percent of the xylose has been converted into ethanol by fermentation. The lignocellulosic biomass consists essentially of corn cobs, corn plant husks, corn plant leaves, and corn stalks.

### FIGURES AND TABLES

FIGURE 1A is a perspective view of a biorefinery comprising a cellulosic ethanol production facility.
FIGURE 1B is a perspective view of a biorefinery comprising a cellulosic ethanol production facility and a corn-based ethanol production facility.
FIGURE 2 is a schematic block diagram of a system for receipt and preparation of biomass for a cellulosic ethanol production facility.
FIGURE 3 is a schematic block diagram of a system for the production of ethanol from biomass.
FIGURES 4A, 4B, and 4C are schematic block diagrams of systems for the treatment of removed components in the production of ethanol from biomass.
FIGURES 5A and 5B are schematic diagrams of the process flow for systems for the production of ethanol from biomass.
FIGURE 6A is a schematic block diagram of an apparatus used for preparation, pre-treatment, and separation of biomass.
FIGURE 6B is a perspective view of an apparatus used to pre-treat and separate the biomass.
FIGURES 7A and 7B are schematic block diagrams of a system for the fermentation of liquid component (C5).
FIGURE 8A is a schematic block diagram of a system for the fermentation of liquid component (C5).
FIGURE 8B is a diagram of a system for the fermentation of liquid component (C5).
FIGURE 9 is a perspective view of an apparatus used for the fermentation liquid component (C5).
FIGURES 10A and 10B are graphs of the results obtained from the fermentation system according to example 1.
FIGURES 11A and 11B are graphs of the results obtained from the fermentation system according to example 2.
FIGURES 12A and 12B are graphs of the results obtained from the fermentation system according to example 3.
TABLES 1A and 1B list the composition of biomass comprising lignocellulosic plant material from the corn plant.
TABLES 2A and 2B list the composition of the liquid component of pre-treated biomass from prepared biomass as indicated in TABLES 1A and 1B.
TABLES 3A and 3B list the composition of the solids component of pre-treated biomass from prepared biomass as indicated in TABLES 1A and 1B.
TABLES 4 through 6 provide data and results obtained from the fermentation system according to example 1, example 2 and example 3.

### DETAILED DESCRIPTION

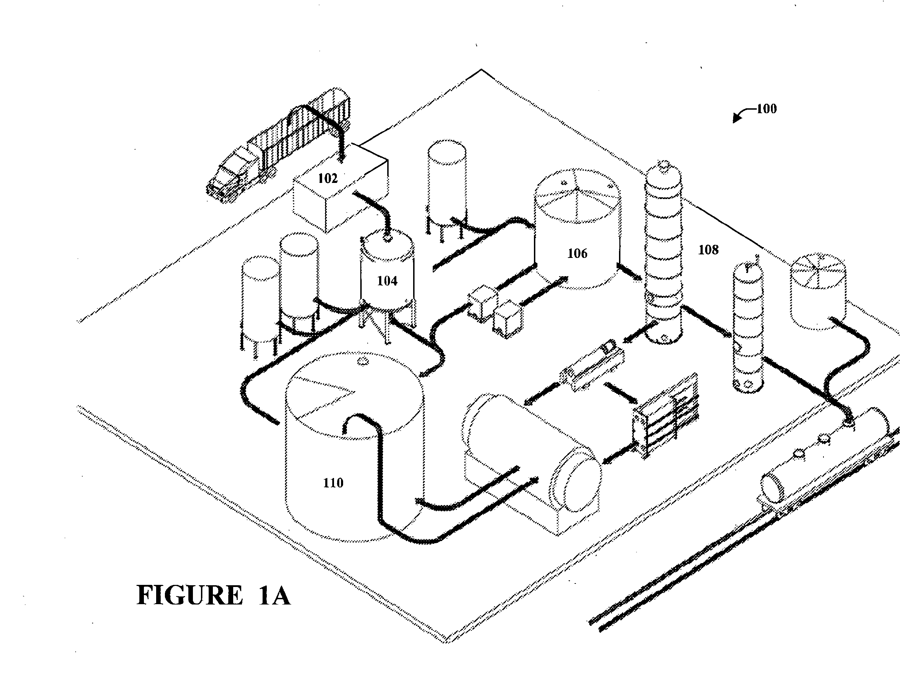

Referring to FIGURE 1A, a biorefinery 100 configured to produce ethanol from biomass is shown.

The biorefinery 100 is configured to produce ethanol from biomass in the form of a lignocellulosic feedstock such as plant material from the corn plant (e.g. corn cobs and corn stover). Lignocellulosic feedstock such as lignocellulosic material from the corn plant comprises cellulose (from which C6 sugars such as glucose can be made available) and/or hemicellulose (from which C5 sugars such as xylose and arabinose can be made available).

As shown in FIGURE 1A, the biorefinery 100 comprises an area where biomass is delivered and prepared to be supplied to the cellulosic ethanol production facility. The cellulosic ethanol production facility comprises apparatus for preparation 102, pre-treatment 104, and treatment of the biomass into treated biomass suitable for fermentation into fermentation product in a fermentation system 106. The facility comprises a distillation system 108 in which the fermentation product is distilled and dehydrated into ethanol. As shown in FIGURE 1A, the biorefinery 100 may also comprise a waste treatment system 110 (shown as comprising an anaerobic digester and a generator). Alternatively, the waste treatment system may comprise other equipment configured to treat, process, and recover components from the cellulosic ethanol production process, such as a solid/waste fuel boiler, anaerobic digester, aerobic digester or other biochemical or chemical reactors.

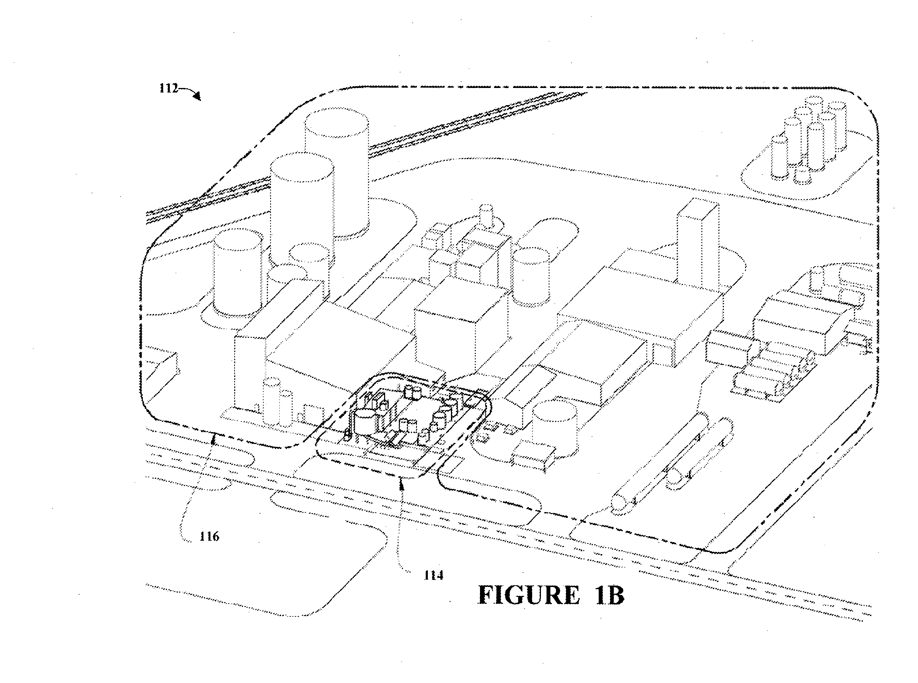

As shown in FIGURE 1B, the biorefinery 112 may comprise a cellulosic ethanol production facility 114 (which produces ethanol from lignocellulosic material and components of the corn plant) co-located with a corn-based ethanol production facility 116 (which produces ethanol from starch contained in the endosperm component of the corn kernel). As indicated in FIGURE 1B, by co-locating the two ethanol production facilities, certain plant systems may be shared, for example, systems for dehydration, storage, denaturing, and transportation of ethanol, energy/fuel-to-energy generation systems, plant management and control systems, and other systems. Corn fiber (a component of the corn kernel), which can be made available when the corn kernel is prepared for milling (e.g. by fractionation) in the corn-based ethanol production facility, may be supplied to the cellulosic ethanol production facility 114 as a feedstock. Fuel or energy sources such as methane or lignin from the cellulosic ethanol production facility 114 may be used to supply power to either or both co-located facilities. Alternatively, a biorefinery (e.g. a cellulosic ethanol production facility) may be co-located with other types of plants and facilities, for example an electric power plant, a waste treatment facility, a lumber mill, a paper plant, or a facility that processes agricultural products.

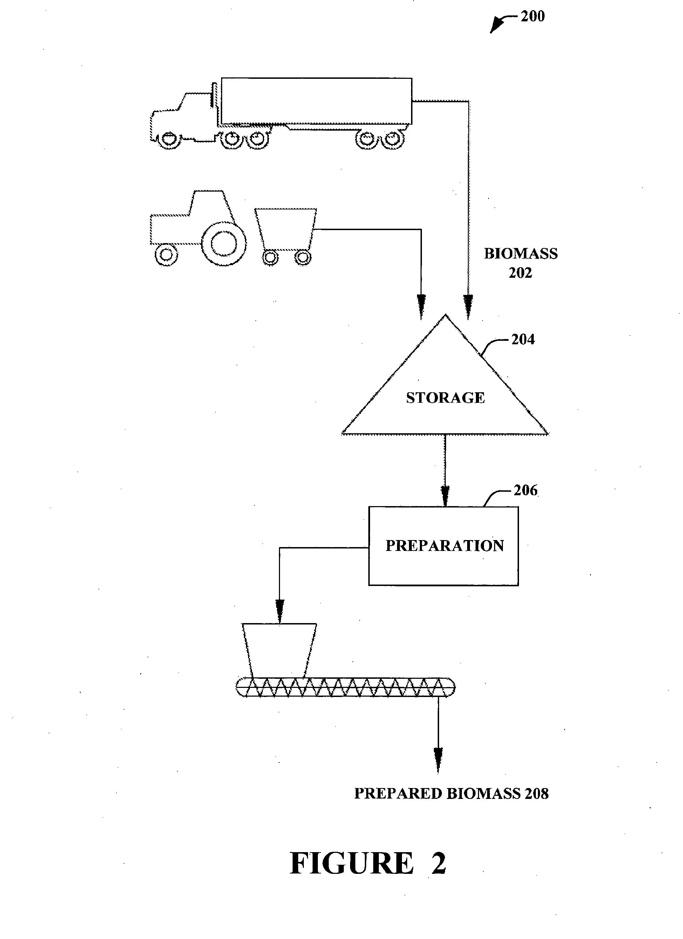

Referring to FIGURE 2, a system 200 for preparation of biomass delivered to the biorefinery is shown. The biomass preparation system 200 may comprise apparatus for receipt/ unloading of the biomass, cleaning (e.g., removal of foreign matter), grinding (e.g., milling, reduction or densification), and transport and conveyance for processing at the plant. The biomass 202 in the form of corn cobs and stover may be delivered to the biorefinery and stored 204 (e.g. in bales, piles or bins, etc.) and managed for use at the facility. The biomass may comprise at least about 20 to about 30 percent corn cobs (by weight) with corn stover and other matter. A preparation system 206 of the biorefinery may be configured to prepare any of a wide variety of types of biomass (e.g., plant material), illustrated as prepared biomass 208, for treatment and processing into ethanol and other bioproducts at the plant.

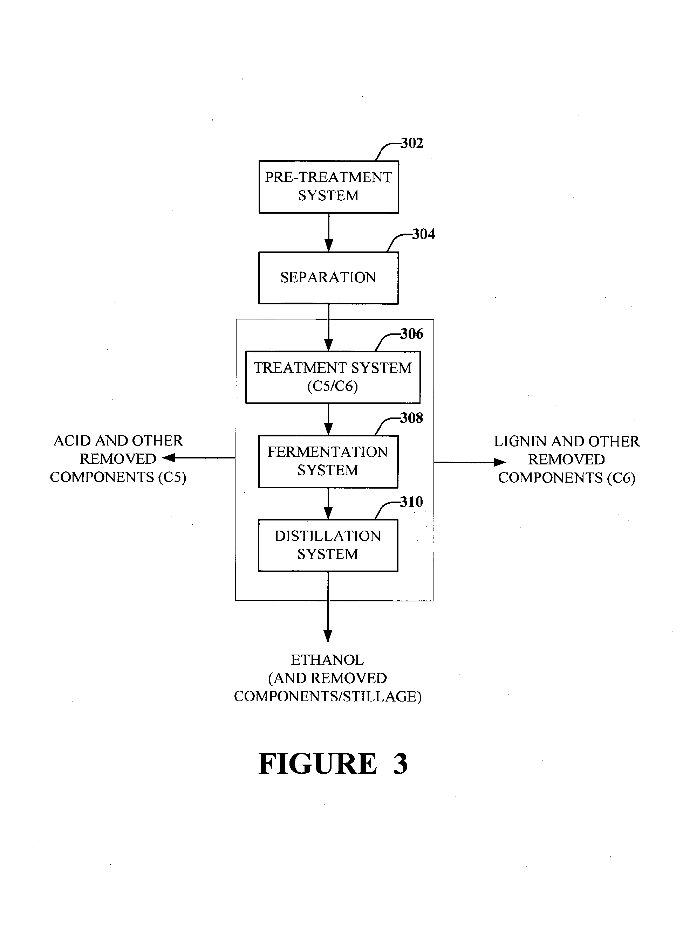

Referring to FIGURE 3, a schematic diagram of the cellulosic ethanol production facility is shown. The biomass comprising plant material from the corn plant is prepared and cleaned at a preparation system. After preparation, the biomass is mixed with water into a slurry and is pre-treated at a pre-treatment system 302. In the pre-treatment system 302, the biomass is broken down (e.g. by hydrolysis) to facilitate separation 304 into a liquid component (e.g. a stream comprising the C5 sugars) and a solids component (e.g. a stream comprising cellulose from which the C6 sugars can be made available). The C5-sugar-containing liquid component (C5 stream) and C6-sugar-containing solids component (C6 stream) can be treated in a treatment system 306 (as may be suitable) and fermented in a fermentation system 308. Fermentation product from the fermentation system 308 is supplied to a distillation system 310 where the ethanol 312 is recovered.

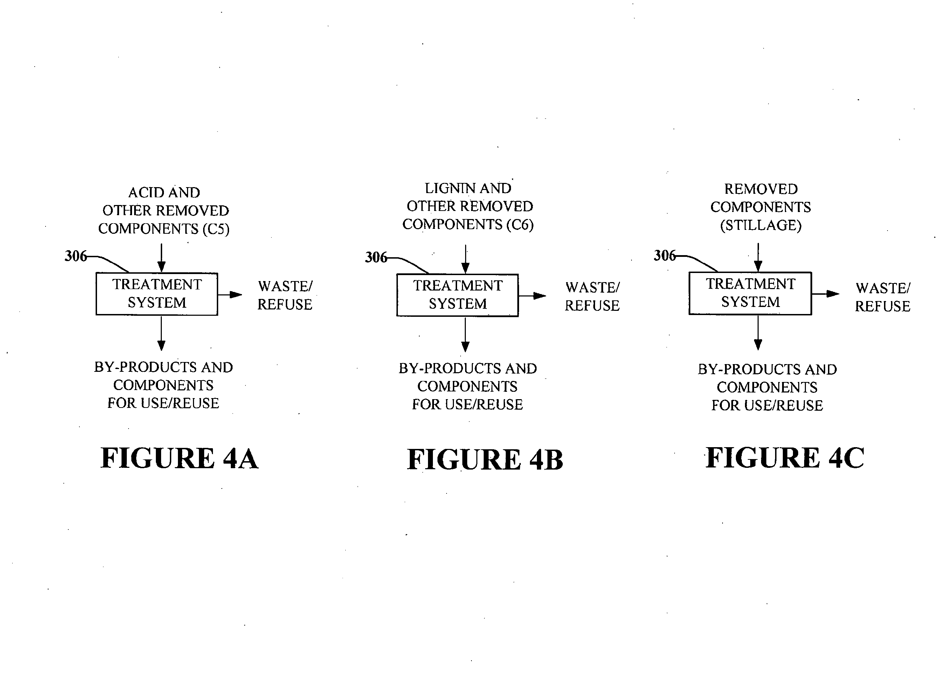

As shown in FIGURES 3 and 4A, 4B and 4C, removed components from treatment (e.g., from the treatment system 306) of the C5 and/or C6 stream can be treated or processed to recover by-products, such as organic acids, furfural and lignin. The removed components during treatment and production of ethanol from the biomass from either or both the C5 stream and the C6 stream (or at distillation) can be treated or processed into bioproducts or into fuel (such as lignin for a solid fuel boiler or methane produced by treatment of residual/removed matter such as acids and lignin in an anaerobic digester) or recovered for use or reuse. As shown in FIGURES 4A, 4B and 4C, components removed during treatment and production of ethanol from the biomass from either or both the C5 stream and the C6 stream (or at distillation) may be processed into bioproducts (e.g. by-products or co-products) or recovered for use or reuse. As shown in FIGURE 4C, removed components from the distillation system (such as stillage or removed solids) or from the treatment of the fermentation product before distillation (e.g. removed solids and particulate matter, which may comprise residual lignin, etc.) can be treated or processed into bioproducts or fuel (e.g. methane produced in an anaerobic digester).

The biomass may comprise plant material from the corn plant, such as corn cobs, husks and leaves and stalks (e.g., at least the upper half or three-quarters portion of the stalk); the composition of the plant material (e.g., cellulose, hemicellulose and lignin) will be approximately as indicated in TABLES 1A and 1B (e.g., after at least initial preparation of the biomass, including removal of any foreign matter). The plant material may comprises corn cobs, husks/leaves and stalks; for example, the plant material may comprise (by weight) up to 100 percent cobs, up to 100 percent husks/leaves, approximately 50 percent cobs and approximately 50 percent husks/leaves, approximately 30 percent cobs and approximately 50 percent husks/leaves and approximately 20 percent stalks, or any of a wide variety of other combinations of cobs, husks/leaves, and stalks from the corn plant. See TABLE 1 A. Alternatively, the lignocellulosic plant material may comprise fiber from the corn kernel (e.g. in some combination with other plant material). TABLE 1B provides typical and expected ranges believed to be representative of the composition of biomass comprising lignocellulosic material from the corn plant. The lignocellulosic plant material of the biomass (from the corn plant) may comprise (by weight) cellulose at about 30 to about 55 percent, hemicellulose at about 20 to about 50 percent, and lignin at about 10 to about 25 percent; particularly, the lignocellulosic plant material of the biomass (e.g., cobs, husks/leaves, and stalk portions from the corn plant) comprises (by weight) cellulose at about 35 to about 45 percent, hemicellulose at about 24 to about 42 percent, and lignin at about 12 to about 20 percent. Particularly, the pre-treatment of the biomass will yield a liquid component that comprises (by weight) xylose at no less than 1.0 percent and a solids component that comprises (by weight) cellulose (from which glucose can be made available) at no less than 45 percent.

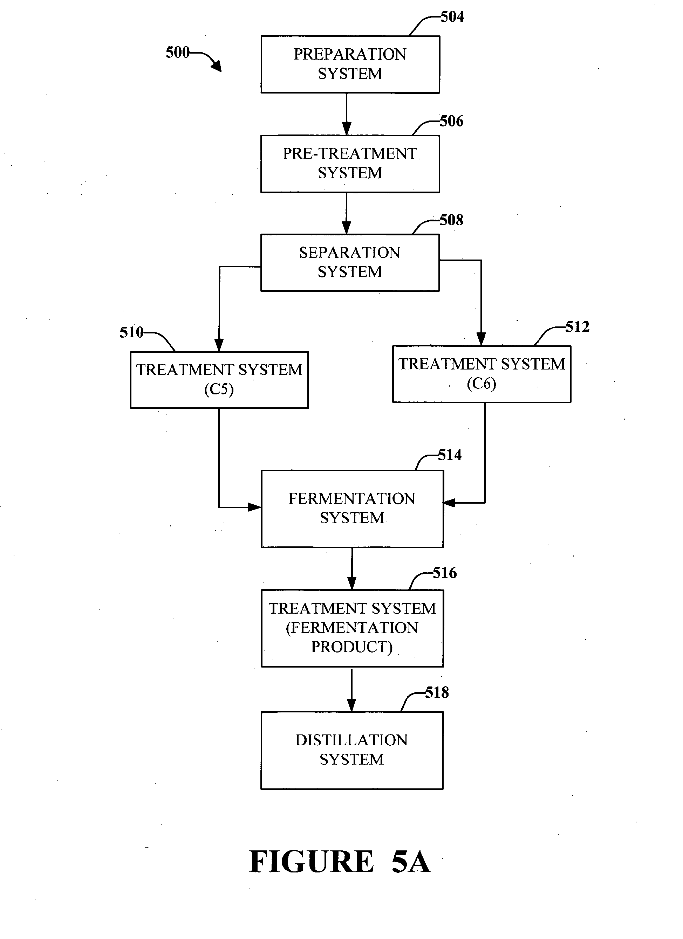

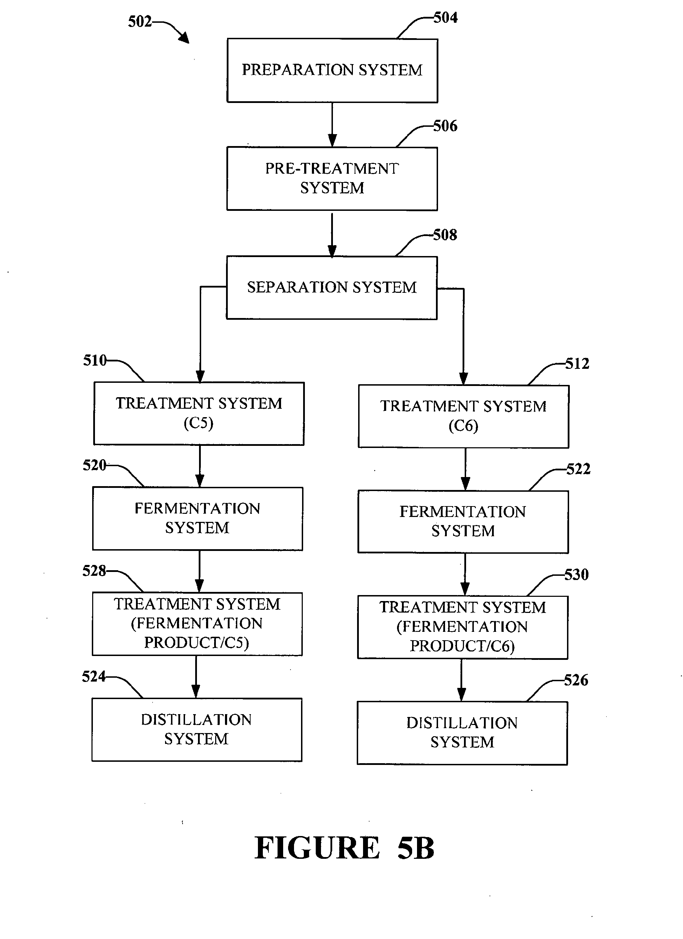

FIGURES 5A and 5B illustrate systems 500, 502 for the production of ethanol from biomass. As shown in FIGURES 5A and 5B, biomass is prepared and cleaned at a preparation system 504 and is pre-treated in a pre-treatment system 506 and then separated (in a separation system 508) into a liquid component and a solids component. After pre-treatment and separation the C5 stream and the C6 stream are processed separately; as shown, the C5 stream and the C6 stream may be processed separately prior to co-fermentation (C5/C6 fermentation as shown in FIGURE 5A) or processed separately including separate fermentation (separate C5 fermentation and C6 fermentation as shown in FIGURE 5B).

Treatment of the C5 stream (liquid component) of the biomass may be performed in an effort to remove components that are inhibitory to efficient fermentation (e.g. furfural, Hydroxymethylfurfural (HMF), sulfuric acid, and acetic acid) and residual lignin (or other matter) that may not be fermentable from the C5 sugar component so that the sugars (e.g. xylose, arabinose, as well as other sugars such as glucose) are available for fermentation. The C5 sugars in the C5 stream may also be concentrated to improve the efficiency of fermentation (e.g. to improve the titer of ethanol for distillation).

Treatment of the C6 stream (solids component) of the biomass may be performed to make the C6 sugars available for fermentation. Hydrolysis (such as enzyme hydrolysis) may be performed to access the C6 sugars in the cellulose; treatment may also be performed in an effort to remove lignin and other non-fermentable components in the C6 stream (or to remove components such as residual acid or acids that may be inhibitory to efficient fermentation).

As shown in FIGURE 5A, after pre-treatment and separation, the C5 stream and the C6 stream can be treated separately (e.g., in separate treatment systems 510, 512) and subsequently combined after treatment (e.g. as a slurry) for co-fermentation in a fermentation system 514 to produce a C5/C6 fermentation product from the available sugars (e.g. xylose and glucose); the C5/C6 fermentation product can (after treatment (in a treatment system 516), if any) be supplied to a distillation system 518 for recovery of the ethanol (e.g. through distillation and dehydration). As shown in FIGURE 5B, the C5 stream and the C6 stream can each be separately processed through fermentation, in fermentation systems 520, 522, and distillation, in distillation systems 524, 526 (after treatment in treatment systems 528, 530, if any) to produce ethanol. A suitable fermenting organism (ethanologen) is used in the fermentation system; the selection of an ethanologen may be based on various considerations, such as the predominant types of sugars present in the slurry. Dehydration and/or denaturing of the ethanol produced from the C5 stream and the C6 stream may be performed either separately or in combination.

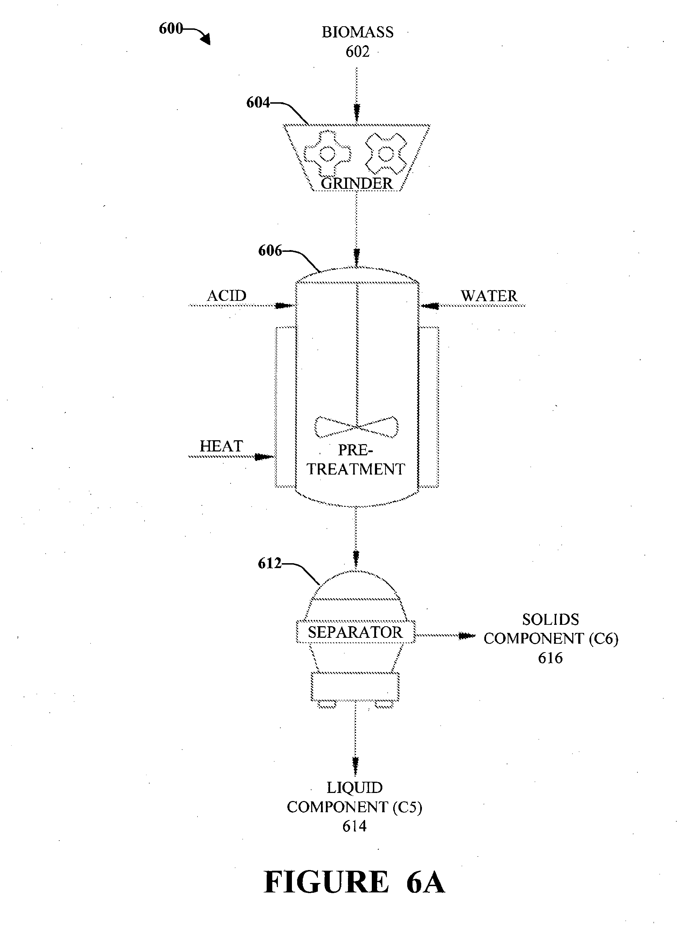

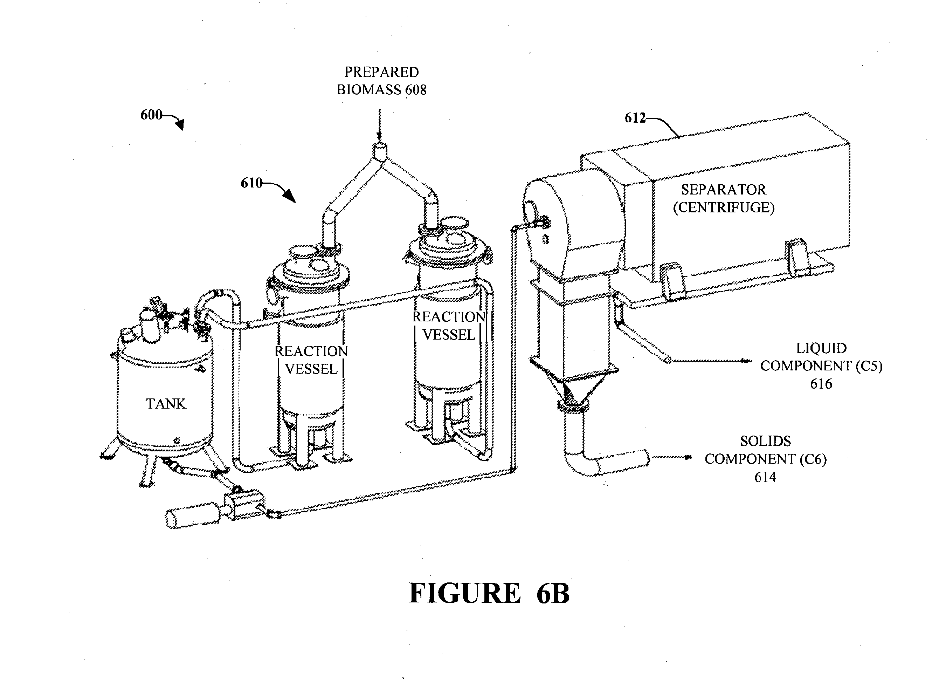

FIGURES 6A and 6B show an apparatus 600 used for preparation, pre-treatment, and separation of lignocellulosic biomass. As shown, biomass 602 is prepared in a grinder 604 (e.g. a grinder or other suitable apparatus or mill). Pre-treatment 606 of the prepared biomass 608 is performed in a reaction vessel (or set of reaction vessels 610) supplied with prepared biomass and acid/water in a predetermined concentration (or pH) and other operating conditions. As shown in FIGURE 6B, the pre-treated biomass can be separated in a centrifuge 612 into a liquid component 614 (C5 stream comprising primarily liquids with some solids) and a solids component 616 (C6 stream comprising liquids and solids such as lignin and cellulose from which glucose can be made available by further treatment).

In the pre-treatment system an acid may be applied to the prepared biomass to facilitate the breakdown of the biomass for separation into the liquid component (C5 stream from which fermentable C5 sugars can be recovered) and the solids component (C6 stream from which fermentable C6 sugars can be accessed). The acid can be applied to the biomass in a reaction vessel under determined operating conditions (e.g., acid concentration, pH, temperature, time, pressure, solids loading, flow rate, supply of process water or steam, etc.) and the biomass can be agitated/mixed in the reaction vessel to facilitate the breakdown of the biomass. An acid such as sulfuric acid, hydrochloric acid, nitric acid, phosphoric acid, acetic acid, etc. (or a formulation/mixture of acids) can be applied to the biomass. Preferably, sulfuric acid will be applied to the biomass in pre-treatment.

The liquid component (C5 stream) comprises water, dissolved sugars (such as xylose, arabinose and glucose) to be made available for fermentation into ethanol, acids, and other soluble components recovered from the hemicellulose. (TABLE 2B provides typical and expected ranges believed to be representative of the composition of biomass comprising lignocellulosic material from the corn plant.) The liquid component may comprise approximately 5 to approximately 7 percent solids (e.g., suspended/residual solids such as partially-hydrolysed hemicellulose, cellulose and lignin). Preferably, the liquid component comprises at least about 2 to about 4 percent xylose (by weight); alternatively, the liquid component comprises no less than approximately 1 to around 2 percent xylose (by weight). TABLES 2A and 2B list the composition of the liquid component of pre-treated biomass (from prepared biomass as indicated in TABLES 1A and 1B).

The solids component (C6 stream) comprises water, acids, and solids such as cellulose from which sugar, such as glucose, can be made available for fermentation into ethanol, and lignin. (TABLE 3B provides typical and expected ranges believed to be representative of the composition of biomass comprising lignocellulosic material from the corn plant.) The solids component may comprise approximately 10 to approximately 40 percent solids (by weight) (after separation); particularly, the solids component will comprise approximately 20 to about 30 percent solids (by weight). The solids in the solids component may comprise no less than around 30 percent cellulose and the solids component may also comprise other dissolved sugars (e.g. glucose and xylose). TABLES 3A and 3B list the composition of the solids component ofpre-treated biomass (from prepared biomass as indicated in TABLES 1A and 1B).

During pre-treatment, the severity of operating conditions (such as pH, temperature, and time) may cause formation of components that are inhibitory to fermentation. For example, under some conditions, the dehydration of sugars (such as xylose or arabinose) may cause the formation of furfural. Acetic acid may also be formed, for example, when acetate is released during the breakdown of hemicellulose in pre-treatment. Sulfuric acid, which may be added to prepared biomass to facilitate pre-treatment, if not removed or neutralized, may also be inhibitory to fermentation. By adjusting pre-treatment conditions (such as pH, temperature, and time), the formation of inhibitors can be reduced or managed; components of the pre-treated biomass may be given further treatment to remove or reduce the level of inhibitors (or other undesirable matter).

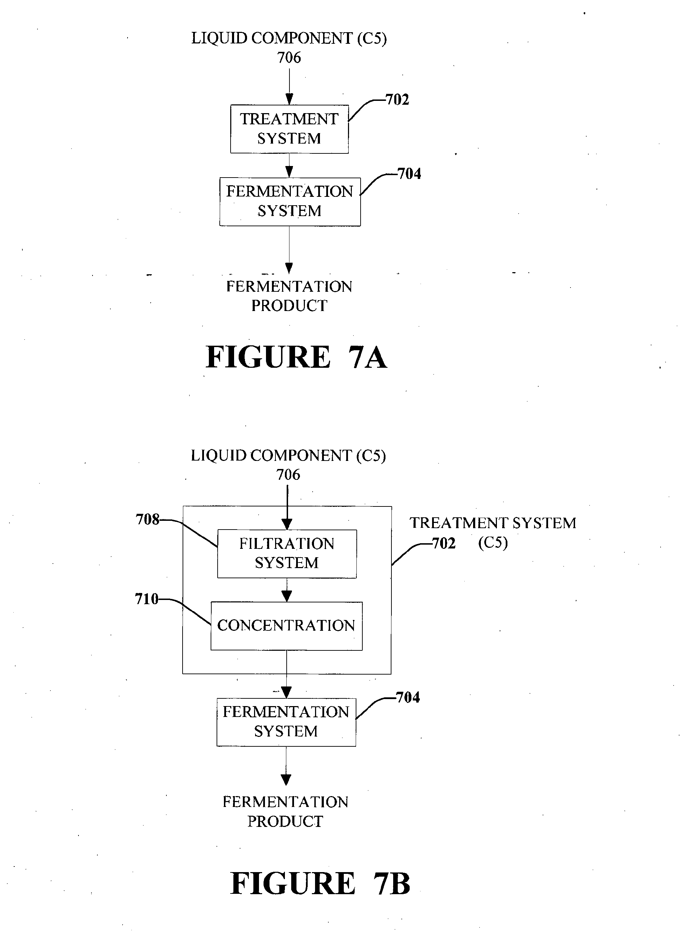

Referring to FIGURES 7A and 7B, a treatment system 702 and fermentation system 704 for the liquid component 706 (C5 stream or hydrolysate) of the pre-treated biomass is shown. As shown in FIGURE 7B, the treatment system 702 can comprise filtration (filtration system 708) of the C5 stream to remove inhibitors (such as furfural and acetic acid) and concentration 710 of the C5 stream to facilitate the efficient fermentation of sugars (e.g. xylose and glucose). An example of a filtration system that can be used to treat the liquid component (C5) is a nanofiltration system.

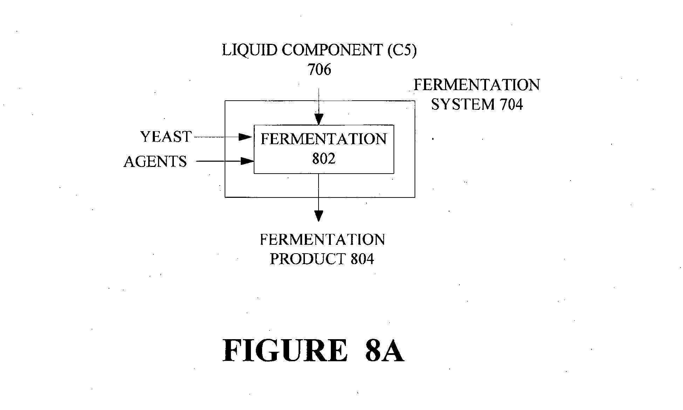

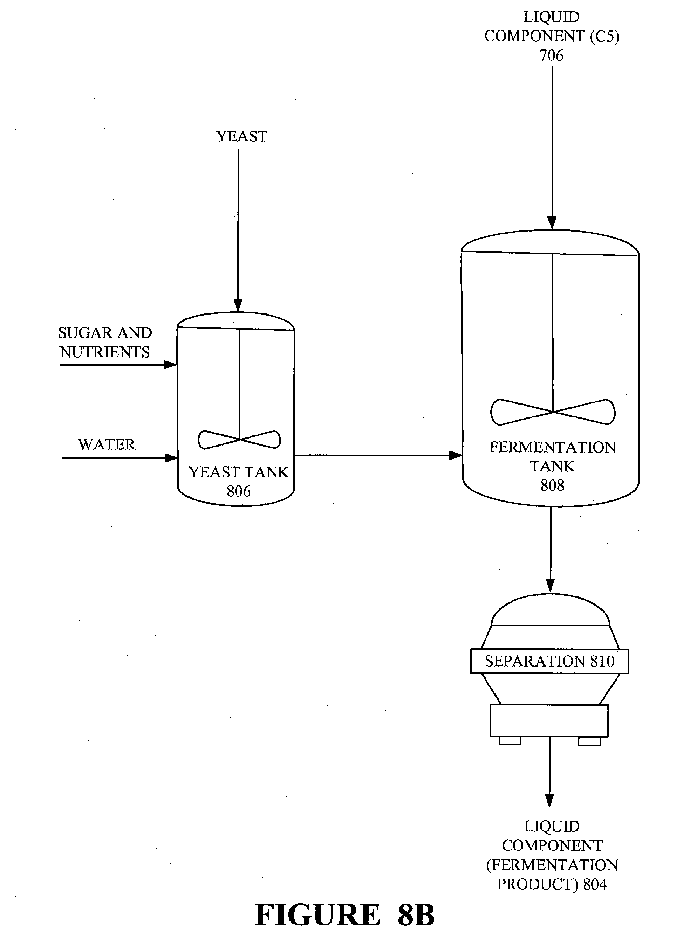

As shown in FIGURES 8A and 8B, the fermentation system 704 can comprise the fermentation 802 of the C5 stream by the application of an ethanologen (e.g., an organism shown as yeast cells) and agents (such as nutrients) for the ethanologen to yield a fermentation product 804. For example, the ethanologen and agents can be combined in a yeast tank 806 and the fermentation can be conducted in a fermentation tank 808. Further to this example, the liquid component (fermentation product 804) is yielded through separation 810.

The fermentation product may be produced in the fermentation system 704 by application of the ethanologen to convert the sugars in the C5 stream (hydrolysate of the pre-treated biomass) into ethanol. The ethanologen for the fermentation system may comprise an organism (e.g., yeast) selected for efficient fermentation of the xylose and glucose that is present in the C5 stream. The ethanologen for the C5 stream may be a genetically modified organism as described in U.S. Patent No. 7,622,284, entitled "Transformed eukaryotic cells that directly convert xylose to xylulose", and assigned to Royal Nedalco B.V. Alternatively, the ethanologen may comprise a formulation or combination of organisms (e.g. one type of yeast selected for fermentation of C5 sugars such as xylose and one type of yeast selected for fermentation of C6 sugars such as glucose). The amount or loading (dose) of ethanologen (e.g., yeast cells) may be varied in the operation of the fermentation system. Agents supplied with the ethanologen may include antibiotics, supplemental or accessory enzymes, urea, salts (such as zinc or magnesium salts), or other components providing nutritional or other benefit to the organism. The agent may comprise thin stillage from a conventional (e.g., corn based) ethanol production facility. The agent may comprise clarified thin stillage, which can be produced from thin stillage by removal of substantially all of solids and oil contained in the thin stillage (e.g. by centrifugation). Clarified thin stillage comprises essentially water and soluble components of thin stillage. The clarified thin stillage may also be concentrated (e.g. by evaporation) prior to its use as an agent in fermentation. Clarified thin stillage may be supplied to the fermentation vessel at around 5 to around 50 percent of the total volume of the slurry. Clarified thin stillage may be supplied to the fermentation vessel at about 15 to about 40 percent of the total volume of the slurry.

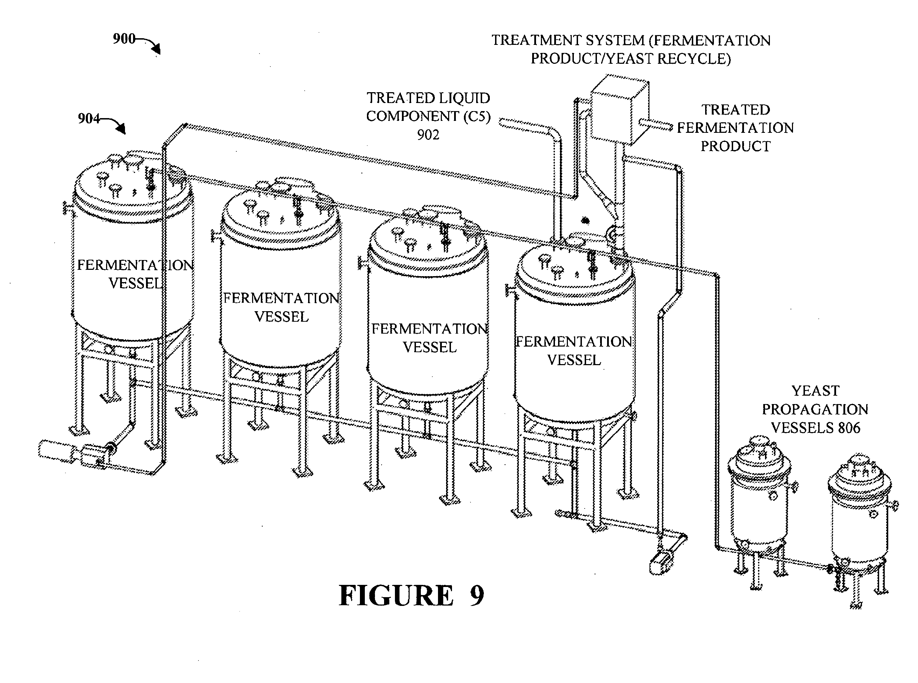

Referring to FIGURE 9, an exemplary apparatus 900 for the fermentation of treated liquid component (C5) 902 is shown. The fermentation system comprises at least one fermentation tank. As shown in FIGURE 9, the fermentation system may comprise a set of tanks (illustrated as fermentation vessels 904) into which the treated C5 stream (e.g., treated hydrolysate from pre-treated biomass, in a slurry) is supplied, along with the ethanologen and nutrients (as needed). As shown in FIGURES 8B and 9, the ethanologen (shown as yeast) is supplied from a yeast propagation system 806 comprising a tank(s) (maintained under operating conditions suitable for growth of a suitable quantity of yeast/organism from seed or source). Fermentation is conducted under operating conditions selected to facilitate the efficient conversion of the sugars in the C5 stream/hydrolysate into ethanol. Operating conditions for the fermentation system comprise time, temperature, pH, solids loading, and ethanologen loading.

The fermentation system may operate in a fed batch arrangement. Fed batch fermentation refers to a process, where instead of supplying all of the substrate into the fermentation vessel at the beginning of fermentation, the substrate is fed into the fermentation vessel gradually throughout fermentation. In some aspects, an initial concentration of substrate is supplied, and more substrate is added either in batches or continuously after some of the initial substrate has been converted into a fermentation product by the fermenting organism. Fed batch fermentation may be used to avoid osmotic stress and thus inhibition of the fermenting organism by high concentrations of substrate. A fed batch fermentation of cellulosic biomass hydrolysate can utilize relatively high cell loadings of the fermenting organism to produce good fermentation results.

Fermenting sugars from cellulosic biomass may be a challenge because of the combination of sugars (e.g. xylose, arabinose, and glucose) that are available for fermentation, and because of the presence of inhibitory components, such as furfural and acetic acid. Commercially available ethanologens (e.g. yeasts) can be optimized to convert glucose to ethanol, and do not efficiently convert other sugars (e.g. xylose and/or arabinose) to ethanol. Fermentation of sugars from cellulosic biomass may be performed by genetically modified strains of fermentation organisms (e.g. as described in U.S. Patent No. 7,622,284, , entitled "Transformed eukaryotic cells that directly convert xylose to xylulose", and assigned to Royal Nedalco B.V.), but the use of such organisms in high cell loading fermentations, such as a fed batch fermentation of C5 sugars (e.g. xylose and/or arabinose), may be economically disadvantageous.

The liquid component (C5) may be fermented in a fed batch arrangement with a low yeast cell loading. For example, the fermentation vessel may be inoculated with approximately 0.1 to approximately 2 grams of yeast (dry weight) per liter of slurry. The fermentation vessel may be_inoculated with about 0.2 to about 1.0 grams of yeast (dry weight) per liter of slurry and preferably, the fermentation vessel is inoculated with about 0.4 to about 0.6 grams of yeast (dry weight) per liter of slurry.

The initial concentration of xylose in the slurry may be in a range of around 1 to around 9 percent of xylose. Preferably, the initial concentration of xylose in the slurry is in a range of about 3 to about 7 percent xylose, and even more preferably, the initial concentration of xylose in the slurry is in a range of around 4.0 to around 5.5 percent xylose. Additional xylose can be fed into the fermentation vessel after about 5 to about 40 hours (or after about 24 to about 36 hours) of fermentation at a rate of approximately 0.5 to approximately 12 grams per liter per hour. Preferably, additional xylose is fed at around 1 to around 8 grams per liter per hour, and more preferably, additional xylose is fed at about 2 to about 5 grams per liter per hour. The total amount of xylose supplied to the fermentation vessel (calculated based on initial xylose and added xylose) may be in a range of approximately 40 to approximately 180 grams per liter of slurry. Preferably, the total amount of xylose supplied to the fermentation vessel is in a range of around 80 to around 160 grams per liter of slurry, and more preferably, the total amount of xylose supplied to the fermentation vessel is in a range of about 110 to about 140 grams per liter of slurry.

The pH of the slurry may be adjusted at the beginning of fermentation to a range of about 4.5 to 6.5, or to a range of about 5.0 to 6.0, or to about 5.5. The fermentation temperature may be held in a range of approximately 25 to 37 degrees Celsius, or in a range of approximately 30 to 33 degrees Celsius, or at about 31.5 to 32.5 degrees Celsius. The total fermentation time may be in a range of about 48 to 168 hours, or in a range of about 72 to 144 hours, or in a range of around 96 to 120 hours. Shorter fermentation times may be achieved by further optimizing the fermentation conditions.

More than 80 percent of the available sugar (e.g. xylose) may be fermented into ethanol. For example, a yield of 83 percent or more may be achieved. For example, if the total amount of xylose added to the slurry is 130 grams per liter, the resulting fermentation product may comprise 7.8 volume percent of ethanol. Alternatively, if 83 grams of xylose per liter of slurry is added, the resulting fermentation product may comprise 5.0 volume percent of ethanol.

The fermentation product (which may also be referred to as beer or fermentation broth, or as comprising beer or fermentation broth) can comprise ethanol and water, as well as unfermented matter (e.g. any unfermented sugars) and non-fermentable matter (e.g. residual lignin and other solids). The fermentation product can also comprise in the form of particulate matter the ethanologen (e.g., yeast cells) that was used to produce ethanol, as well as other components produced by the fermentation system, for example, such as glycerol (a product of fermentation) and acetic acid.

As shown in FIGURES 2, 5A and 5B, the liquid component (or treated fermentation product) from the treatment system can be supplied to the distillation system, for distillation and dehydration to allow recovery of ethanol.

A series of examples were conducted using the system shown in FIGURES 8A and 8B in an effort to determine operating conditions for fed batch fermentation of liquid component (C5). The liquid component (C5) was prepared by pre-treating ground corn cobs at 120 degrees Celsius for 2 hours in a 1 percent (w/w) sulfuric acid solution and by centrifuging and filtering to remove any remaining solids. The pre-treated liquid component (C5) was treated by nanofiltration to remove inhibitory components and to concentrate the sugars (e.g. xylose) to about 9 percent (w/v). The ethanologen used in the examples was a strain of Saccharomyces cerevisiae yeast altered to convert xylose and glucose to ethanol (a genetically modified yeast derived from an organism as described in U.S. Patent No. 7,622,284 by Royal Nedalco B.V., for example strain No. RN1016). The treated liquid component was supplied to the fermentation vessels to result in an initial xylose concentration of about 4.9 percent (weight per volume). Thin stillage from a no-cook corn fermentation process was clarified by removing the solids to produce clarified thin stillage. The clarified thin stillage was added to the fermentation vessels at about 36 percent of the volume of the slurry to provide nutrients for the yeast. Urea was also added at a concentration of 0.24 grams per liter of slurry. The fermentation vessels were inoculated with approximately 0.5 grams of yeast (dry weight) per liter of slurry, and the initial pH of the slurry was adjusted to 5.5. The fermentations were carried out at 32 degrees Celsius, and the slurry was agitated continuously during fermentation. Data from the examples is shown in TABLES 4 through 6.

### Example 1

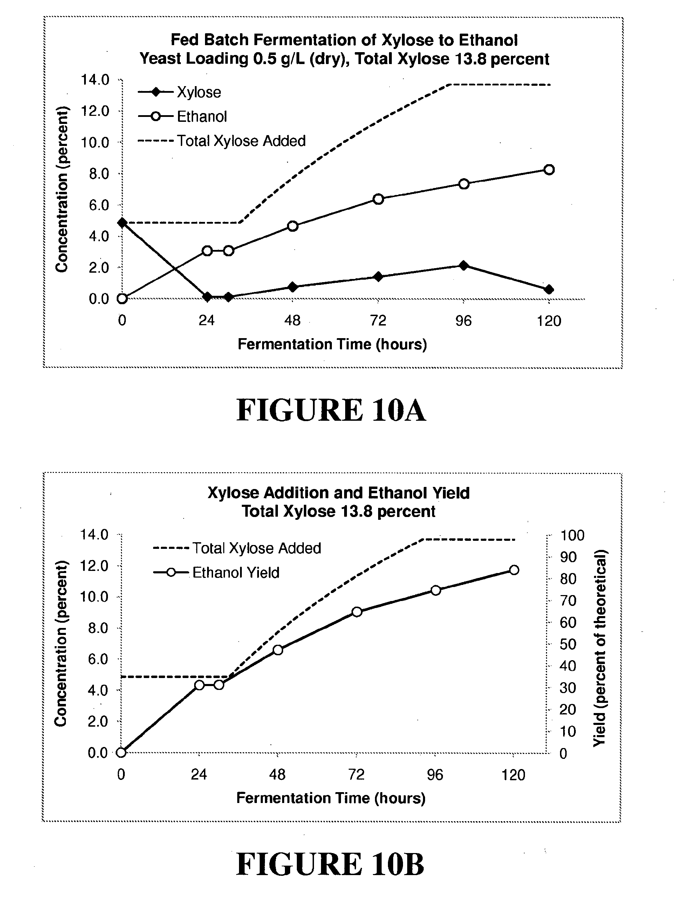

The fermentation system was used in Example 1 to determine suitable operating conditions for fed batch fermentation of liquid component (C5). After 30 hours of fermentation, additional xylose was fed into the fermentation vessel in the form of treated liquid component (C5) at a rate of about 3.0 grams of xylose per liter of slurry per hour for about 65 hours. The fermentation was carried out for a total of 120 hours, and samples of the slurry were tested for xylose and ethanol concentration periodically. The total amount of xylose supplied to the fermentation vessel calculated from the initial xylose and added xylose was 13.8 percent (w/v). It was observed that an ethanol concentration of 8.3 percent (v/v) and a final yield of 84 percent of theoretical yield could be achieved. It was also observed that an improved fermentation yield as compared to previously known methods could be achieved by using the described fermentation conditions. The results from Example 1 are shown in FIGURES 10A and 10B and TABLE 4.

### Example 2

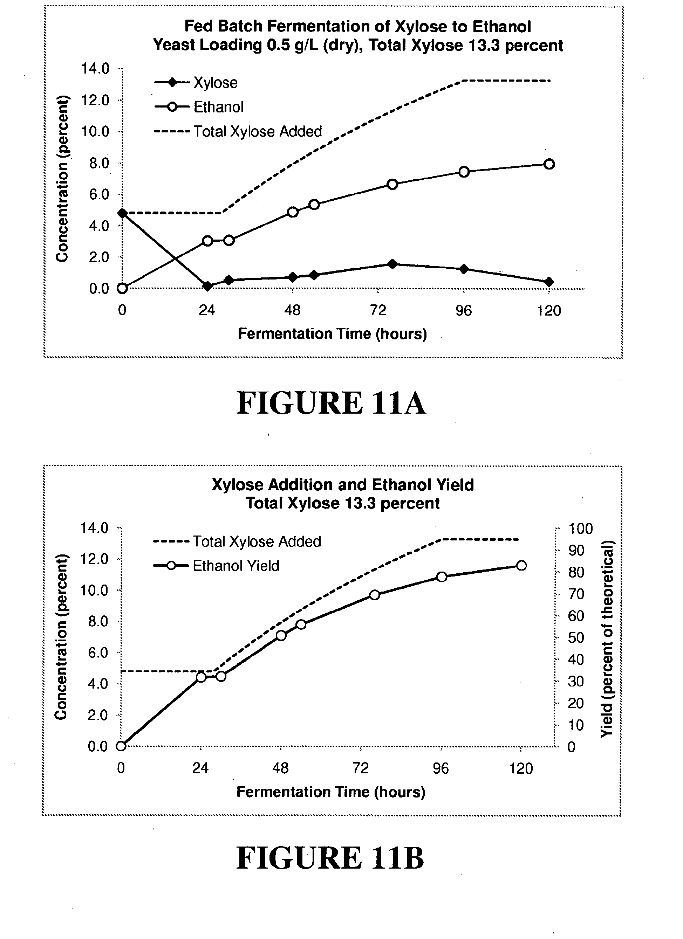

The fermentation system was used in Example 2 to determine suitable operating conditions for fed batch fermentation of liquid component (C5). After 24 hours of fermentation, additional xylose was fed into the fermentation vessel in the form of treated liquid component (C5) at a rate of about 2.4 grams of xylose per liter of slurry per hour for about 72 hours. The fermentation was carried out for a total of 120 hours, and samples of the slurry were tested for xylose and ethanol concentration periodically. The total amount of xylose supplied to the fermentation vessel calculated from the initial xylose and added xylose was 13.3 percent (w/v). It was observed that an ethanol concentration of 8.0 percent (v/v) and a final yield of 83 percent of theoretical yield could be achieved. It was also observed that an improved fermentation yield as compared to previously known methods could be achieved by using the described fermentation conditions. The results from Example 2 are shown in FIGURES 11A and 11B and TABLE 5.

### Example 3

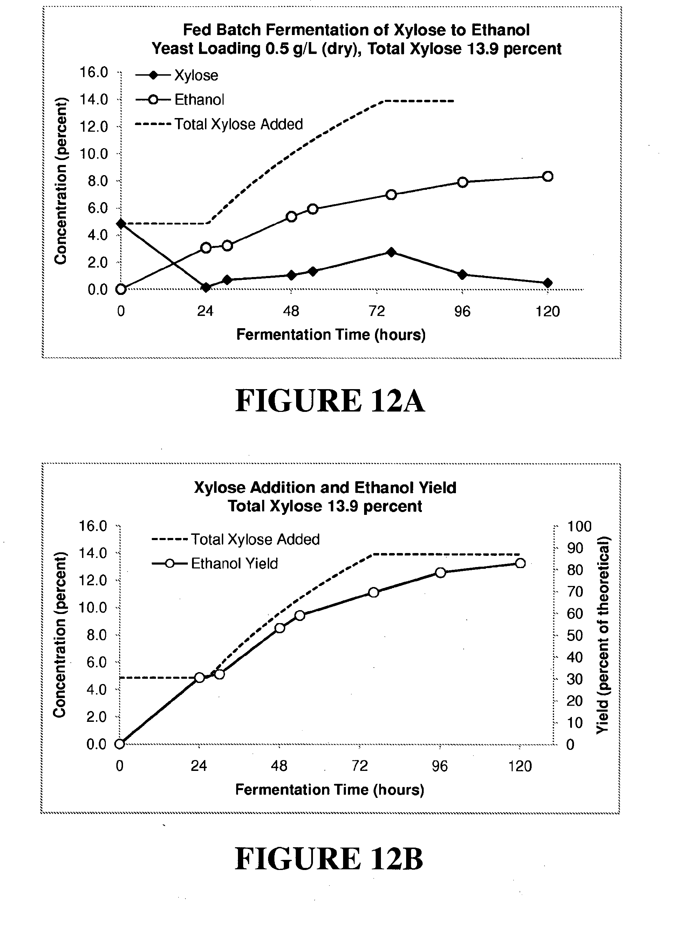

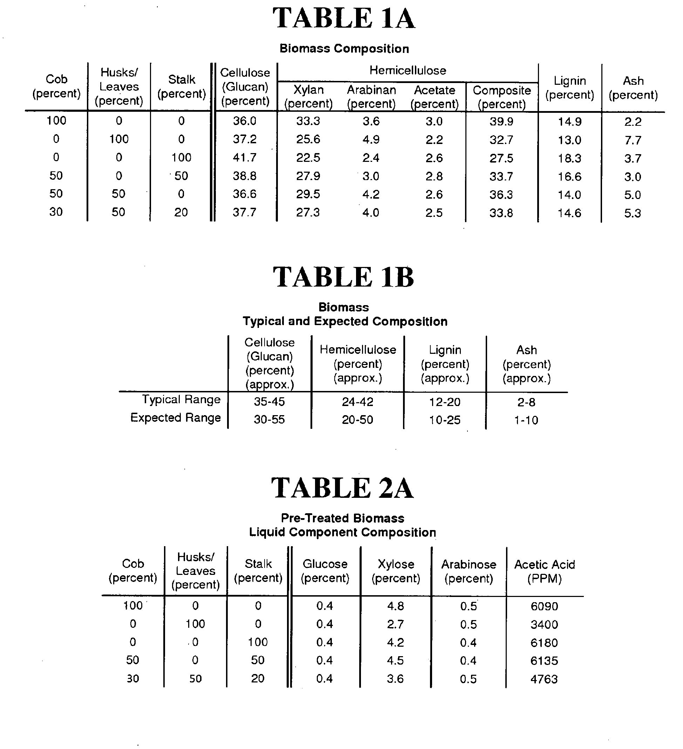

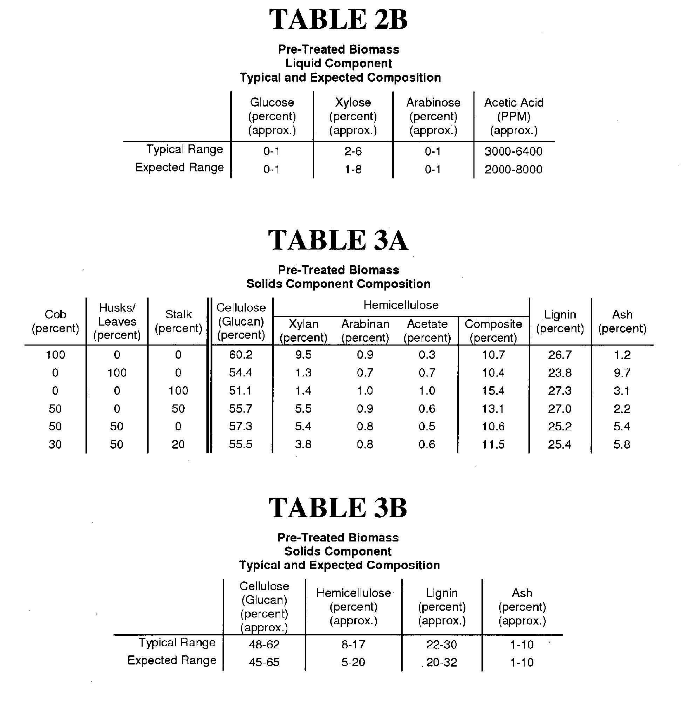

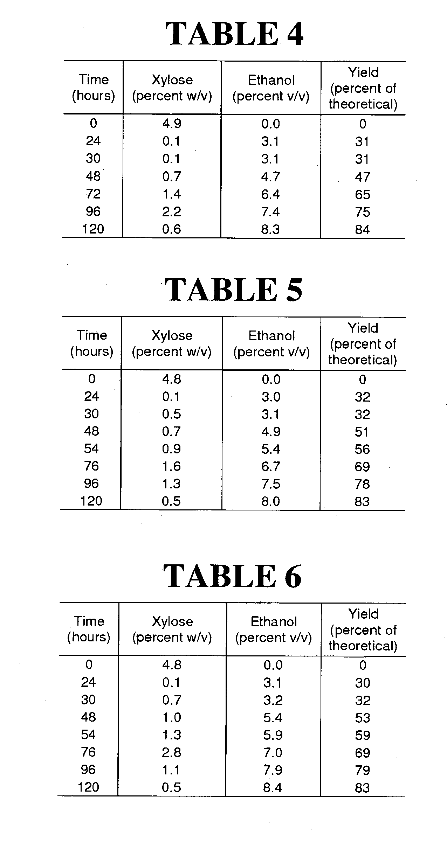

The fermentation system was used in Example 3 to determine suitable operating conditions for fed batch fermentation of liquid component (C5). After 24 hours of fermentation, additional xylose was fed into the fermentation vessel in the form of treated liquid component (C5) at a rate of about 3.68 grams of xylose per liter of slurry per hour for about 52 hours. The fermentation was carried out for a total of 120 hours, and samples of the fermentation broth were tested for xylose and ethanol concentration periodically. The total amount of xylose supplied to the fermentation vessel calculated from the initial xylose and added xylose was 13.9 percent (w/v). It was observed that an ethanol concentration of 8.4 percent (v/v) and a final yield of 83 percent of theoretical yield could be achieved. It was also observed that an improved fermentation yield as compared to previously known methods could be achieved by using the described fermentation conditions. The results from Example 3 are shown in FIGURES 12A and 12B and TABLE 6.

The term "or" is intended to mean an inclusive "or" rather than an exclusive "or." To the extent that the terms "comprises," "has," "contains," and other similar words are used in either the detailed description or the claims, for the avoidance of doubt, such terms are intended to be inclusive in a manner similar to the term "comprising" as an open transition word without precluding any additional or other elements.

## Claims

1. A method for producing a fermentation product in a fermentation system from biomass comprising lignocellulosic material that has been pre-treated and separated into a liquid component and a solid component, the method comprising:
preparing a slurry comprising:
supplying the liquid component comprising pentose to the fermentation system, wherein the pentose comprises xylose;
providing an ethanologen to the fermentation system, wherein the ethanologen is capable of fermenting xylose into ethanol, further wherein the ethanologen is supplied to the fermentation system in a concentration of less than 2 grams of ethanologen on a dry basis per liter of slurry;
adjusting a pH of the slurry to a range of 4.5 to 6.5;
maintaining the liquid component and ethanologen in the fermentation system at a temperature of between 25 and 37 degrees Celsius;
supplying an additional amount of the liquid component to the slurry, wherein the liquid component comprises xylose and wherein the supplying the additional amount of the liquid component begins at least 5 hours, but no more than 40 hours after providing the ethanologen to the slurry; and
recovering the fermentation product from the fermentation system.

2. The method of Claim 1, wherein the initial concentration of xylose in the slurry is of 1 to 9 percent by weight.

3. The method of Claims 1 or 2, wherein the supplying the additional amount of the liquid component is at a rate of 0.5 to 12 grams of xylose added per liter of slurry per hour.

4. The method of any one of Claims 1 - 3, wherein a total amount of xylose added to the slurry is between 40 to 180 grams per liter of slurry.

5. The method of any one of Claims 1 - 4, further comprising supplying an agent to the slurry, wherein the agent comprises thin stillage.

6. The method of any one of Claims 1 - 5, further comprising supplying an agent to the slurry, wherein the agent comprises clarified thin stillage comprising 5 to 50 percent of the volume of the slurry.

7. The method of any one of Claims 1 - 6, wherein the biomass comprises hemi-cellulose and the fermentation product has been produced by fermentation of xylose and wherein the liquid component comprises xylose and glucose and the ethanologen comprises an organism capable of fermenting xylose into ethanol and glucose into ethanol.

8. The method of any one of Claims 1 - 7, wherein the biomass comprises hemi-cellulose and the fermentation product has been produced by fermentation of xylose and wherein the ethanologen is a yeast and the ethanologen comprises Saccharomyces cerevisiae.

9. The method of any one of Claims 1 - 8, wherein the fermentation product comprises ethanol, and wherein at least 75 percent of the xylose has been converted into ethanol by fermentation.

10. The method of any one of Claims 1 - 9, further comprising:
treating the liquid component to reduce inhibitors or to increase a concentration of xylose.

11. The method of any one of Claims 1 - 10, wherein the maintaining comprises maintaining the liquid component and the ethanologen in the fermentation system for a time of no less than 48 hours.

12. A method for producing a fermentation product in a fermentation system from lignocellulosic biomass that has been pre-treated and separated into a liquid component and a solid component, the method comprising:
preparing a slurry comprising:
supplying the liquid component to the fermentation system;
providing an ethanologen to the fermentation system in a concentration of less than 2 grams of ethanologen on a dry basis per liter of slurry, wherein the ethanologen is capable of fermenting xylose into ethanol;
adjusting a pH of the slurry to a range of 4.5 to 6.5;
maintaining the liquid component and the ethanologen in the fermentation system at a temperature of between 25 and 37 degrees Celsius for a duration of at least 48 hours;
supplying an additional amount of the-liquid component to the slurry, wherein the liquid component comprises xylose and wherein the supply of the additional amount of the liquid component begins at least 5 hours but no more than 40 hours after providing the ethanologen to the slurry, wherein the additional amount of the liquid component is supplied to the slurry at a rate of 2 to 5 grams of xylose added per liter of slurry per hour, and wherein a total amount of xylose added to the slurry is between 110 to 140 grams per liter of slurry;
recovering the fermentation product from the fermentation system, wherein the fermentation product comprises ethanol, and wherein at least 75 percent of the xylose has been converted into ethanol by fermentation; and
wherein the lignocellulosic biomass consists essentially of corn cobs, corn plant husks, corn plant leaves, and corn stalks.

## Patentansprüche

1. Ein Verfahren zur Herstellung eines Fermentationsprodukts in einem Fermentationssystem aus Biomasse umfassend Lignocellulosematerial, das vorbehandelt und in eine flüssige Komponente und eine feste Komponente getrennt wurde, wobei das Verfahren:
das Herstellen einer Aufschlämmung umfassend:
Zuführen der flüssigen Komponente, welche Pentose umfasst, in das Fermentationssystem, wobei Pentose Xylose umfasst;
Bereitstellen eines Ethanologens in dem Fermentationssystem, wobei das Ethanologen fähig ist, Xylose in Ethanol zu fermentieren, wobei ferner das Ethanologen dem Fermentationssystem in einer Konzentration von weniger als 2 Gramm Ethanologen bezogen auf eine trockene Basis pro Liter Aufschlämmung zugeführt wird;
Einstellen des pH-Wertes der Aufschlämmung auf einen Bereich von 4,5 bis 6,5; Halten der flüssigen Komponente und des Ethanologens bei einer Temperatur im Bereich von 25 bis 37 Grad Celsius im Fermentationssystem;
Zuführen einer zusätzlichen Menge der flüssigen Komponente zu der Aufschlämmung, wobei die flüssige Komponente Xylose umfasst und wobei das Zuführen der zusätzlichen Menge von der flüssigen Komponente zumindest 5 Stunden, aber nicht später als 40 Stunden nach Bereitstellung des Ethanologen zu der Aufschlämmung erfolgt; und
die Gewinnung des Fermentationsprodukts aus dem Fermentationssystem
umfasst.

2. Das Verfahren gemäß Anspruch 1, wobei die initiale Konzentration von Xylose in der Aufschlämmung 1 bis 9 Gewichtsprozent beträgt.

3. Das Verfahren gemäß einem der Ansprüche 1 oder 2, wobei das Zuführen der zusätzlichen Menge der flüssigen Komponente bei einer Rate von 0,5 bis 12 Gramm Xylose pro zugegebenem Liter der Aufschlämmung pro Stunde stattfindet.

4. Das Verfahren gemäß einem der Ansprüche 1 bis 3, wobei eine Menge an Xylose, die insgesamt zu der Aufschlämmung hinzugegeben wird, zwischen 40 bis 180 Gramm pro Liter Aufschlämmung beträgt.

5. Das Verfahren gemäß einem der Ansprüche 1 bis 4, zusätzlich umfassend Zuführen eines Mittels zu der Aufschlämmung, wobei das Mittel Dünnschlempe umfasst.

6. Das Verfahren gemäß einem der Ansprüche 1 bis 5, zusätzlich umfassend Zuführen eines Mittels zu der Aufschlämmung, wobei das Mittel aufgereinigte Dünnschlempe umfasst, welche 5 bis 50 Volumenprozent der Aufschlämmung umfasst.

7. Das Verfahren gemäß einem der Ansprüche 1 bis 6, wobei die Biomasse Hemizellulose und das Fermentationsprodukt umfasst, welches durch Fermentation von Xylose hergestellt wurde, und wobei die flüssige Komponente Xylose und Glucose umfasst und das Ethanologen einen Organismus fähig zur Fermentierung von Xylose in Ethanol und Glucose in Ethanol umfasst.

8. Das Verfahren gemäß einem der Ansprüche 1 bis 7, wobei die Biomasse Hemizellulose umfasst und das Fermentationsprodukt durch Fermentation von Xylose hergestellt wurde, und wobei das Ethanologen eine Hefe ist und das Ethanologen Saccharomyces cerevisiase umfasst.

9. Das Verfahren gemäß einem der Ansprüche 1 bis 8, wobei das Fermentationsprodukt Ethanol umfasst, und wobei mindestens 75 Prozent der Xylose durch Fermentation in Ethanol umgewandelt wurde.

10. Das Verfahren gemäß einem der Ansprüche 1 bis 9, zusätzlich umfassend:
Behandlung der flüssigen Komponente um Inhibitoren zur reduzieren oder um eine Konzentration von Xylose zu erhöhen.

11. Das Verfahren gemäß einem der Ansprüche 1 bis 10, wobei das Halten umfasst Halten der flüssigen Komponente und des Ethanologens in dem Fermentationssystem nicht weniger als 48 Stunden beträgt.

12. Ein Verfahren zur Herstellung eines Fermentationsprodukts in einem Fermentationssystem aus lignocellulosehaltiger Biomasse, welche vorbehandelt und in eine flüssige Komponente und eine feste Komponente getrennt wurde, wobei das Verfahren:
das Herstellen einer Aufschlämmung, umfassend:
Zuführen der flüssigen Komponente in das Fermentationssystem;
Bereitstellen eines Ethanologens in dem Fermentationssystem in einer Konzentration von weniger als 2 Gramm Ethanologen bezogen auf eine trockene Basis pro Liter Aufschlämmung, wobei das Ethanologen fähig ist Xylose in Ethanol zu fermentieren;
Einstellen des pH-Wertes der Aufschlämmung auf einen Bereich von 4,5 bis 6,5; Halten der flüssigen Komponente und des Ethanologens bei einer Temperatur im Bereich von 25 bis 37 Grad Celsius für eine Dauer von mindestens 48 Stunden im Fermentationssystem;
Zuführen einer zusätzlichen Menge der flüssigen Komponente zu der Aufschlämmung, wobei die flüssige Komponente Xylose umfasst und wobei das Zuführen der zusätzlichen Menge von der flüssigen Komponente zumindest 5 Stunden, aber nicht später als 40 Stunden nach Bereitstellung des Ethanologen zu der Aufschlämmung erfolgt, wobei die zusätzliche Menge der flüssigen Komponente bei einer Rate von 0,5 bis 12 Gramm Xylose pro zugegebenem Liter der Aufschlämmung pro Stunde zugegeben wird, und wobei eine Menge an Xylose die insgesamt zu der Aufschlämmung hinzugegeben wird zwischen 110 bis 140 Gramm pro Liter Aufschlämmung beträgt;
Gewinnung des Fermentationsprodukts aus dem Fermentationssystem, wobei
das Fermentationsprodukt Ethanol umfasst, und wobei mindestens 75 Prozent der Xylose durch Fermentation in Ethanol umgewandelt wurde; und wobei die lignocellulosehaltige Biomasse hauptsächlich Maiskolben, Maispflanze, Hülsen, Blätter der Maispflanze und Maisstroh umfasst.

## Revendications

1. Une méthode pour la production d'un produit de fermentation dans un système de fermentation à partir de la biomasse comprenant un matériel lignocéllulosique, qui a été prétraité et séparé en un composant liquide et un composant solide, la méthode comprenant:
préparer une boue comprenant:
alimenter le composant liquide comprenant du pentose au système de fermentation, où le pentose comprend du xylose;
fournir un éthanologène au système de fermentation, où l'éthanologène est capable de fermenter le xylose en éthanol, où de plus l'éthanologène est fourni au système de fermentation à une concentration de moins de 2 grammes d'éthanologène à sec par litre de boue;
ajuster le pH de la boue dans une gamme de 4,5 à 6,5;
maintenir le composant liquide et l'éthanologène dans le système de fermentation à une température comprise entre 25 et 37 degrés Celsius;
alimenter une quantité supplémentaire de composant liquide à la boue, où le composant liquide comprend du xylose et où l'alimentation de la quantité supplémentaire de composant liquide commence au moins 5 heures, mais pas plus tard que 40 heures après avoir fourni l'éthanologène à la boue; et
récupérer le produit de fermentation à partir du système de fermentation.

2. La méthode selon la revendication 1, où la concentration initiale en xylose dans la boue est de 1 à 9 pourcent par poids.

3. La méthode selon les revendications 1 ou 2, où l'alimentation de la quantité supplémentaire de composant liquide se fait à un taux de 0,5 à 12 grammes de xylose ajoutée par litre de boue par heure.

4. La méthode selon une quelconque des revendications 1 - 3, où la quantité totale de xylose ajoutée à la boue est comprise entre 40 et 180 grammes par litre de boue.

5. La méthode selon une quelconque des revendications 1 - 4, comprenant de plus: fournir un agent à la boue, où l'agent comprend des résidus de distillation.

6. La méthode selon une quelconque des revendications 1 - 5, comprenant de plus: fournir un agent à la boue, où l'agent comprend des résidus de distillation de clarification comprenant de 5 à 50 pourcent du volume de la boue.

7. La méthode selon une quelconque des revendications 1 - 6, où la biomasse comprend de l'hémicellulose et le produit de fermentation a été produit par fermentation du xylose, et où le composant liquide comprend du xylose et du glucose, et l'éthanologène comprend un organisme capable de fermenter le xylose en éthanol et le glucose en éthanol.

8. La méthode selon une quelconque des revendications 1 - 7, où la biomasse comprend de l'hémicellulose et le produit de fermentation a été produit par fermentation du xylose, et où l'éthanologène est une levure et l'éthanologène comprend Saccharomyces cerevisiae.

9. La méthode selon une quelconque des revendications 1 - 8, où le produit de fermentation comprend de l'éthanol, et où au moins 75 pourcent du xylose a été converti en éthanol par fermentation.

10. La méthode selon une quelconque des revendications 1 - 9, comprenant de plus: traiter le composant liquide pour réduire les inhibiteurs ou pour augmenter la concentration en xylose.

11. La méthode selon une quelconque des revendications 1 - 10, où la maintenance comprend maintenir le composant liquide et l'éthanologène dans le système de fermentation pour une durée d'au moins 48 heures.

12. Une méthode pour la production d'un produit de fermentation dans un système de fermentation à partir de la biomasse lignocéllulosique, qui a été prétraitée et séparée en un composant liquide et un composant solide, la méthode comprenant :
préparer une boue comprenant:
alimenter le composant liquide au système de fermentation;
fournir au système de fermentation un éthanologène à une concentration de moins de 2 grammes d'éthanologène à sec par litre de boue, où l'éthanologène est capable de fermenter le xylose en éthanol;
ajuster le pH de la boue dans une gamme de 4,5 à 6,5;
maintenir le composant liquide et l'éthanologène dans le système de fermentation à une température comprise entre 25 et 37 degrés Celsius pour une durée d'au moins 48 heures;
alimenter une quantité supplémentaire de composant liquide à la boue, où le composant liquide comprend du xylose et où l'alimentation de la quantité supplémentaire de composant liquide commence au moins 5 heures, mais pas plus tard que 40 heures après avoir fourni l'éthanologène à la boue, où la quantité supplémentaire de composant liquide est ajoutée à la boue à un taux de 2 à 5 grammes de xylose ajouté par litre de boue par heure, et où une quantité totale de xylose ajoutée à la boue est comprise entre 110 et 140 grammes par litre de boue;
récupérer le produit de fermentation du système de fermentation, où le produit de fermentation comprend de l'éthanol, et où au moins 75 pourcent du xylose a été converti en éthanol par fermentation; et
où la biomasse lignocéllulosique consiste essentiellement en épis de maïs, enveloppes de la plante de maïs, feuilles de la plante de maïs et tiges de maïs.
